# EUROPEAN PATENT APPLICATION

(11) **EP 4 310 499 A1**
(43) Date of publication of application: **24.01.2024**
(21) Application number: 23186447.1
(22) Date of filing: 19.07.2023
(51) Int. Cl.: G01N 33/543

(54) **BIOLOGICAL DETECTION CHIP AND APPLICATION THEREOF**

(30) Priority: 19.07.2022 US 202263390347 P
(71) Applicant: Sunplus Technology Co., Ltd, Hsinchu 300 (TW); Kao, Hsi-Teng, Hsinchu City 300 (TW)
(72) Inventor: CHIOU, Ming-Tang, 900 Pingtung City, Pingtung County (TW); LIN, Wei-Hao, 906 Pingtung County (TW); KAO, Hsi-Teng, 300 Hsinchu City (TW); CHENG, Daisy, 241 New Taipei City (TW); LIN, Shi-Wei, 406 Taichung City (TW); LEE, Yi-Chan, 300 Taoyuan City (TW)
(74) Representative: Wittmann, Günther

(57) **Abstract**

The present invention disclosed a biological detection chip and its application thereof. The biological detection chip includes a plurality of transistors in parallel (an array having plurality of rows and a plurality of columns of transistors). Each of the transistors includes a substrate layer comprising a shared source, a shared drain, and a channel area disposed between the shared source and the shared drain, a floating gate, an extending metal connector, an extending gate, and a biological detection layer on the extending gate. The biological detection chip is combined with biological probes after the surface modification process for biological detection. Biological detection includes Porcine Reproductive and Respiratory Syndrome Virus (PRRSV), Porcine Epidemic Diarrhea Virus (PEDV), and Cortisol detection.

## Description

### CROSS-REFERENCE TO RELATED APPLICATIONS

This application claims priority to and the benefit of U.S. Provisional Application No. 63/390,347, filed on July 19, 2022, the disclosure of which is incorporated by reference in its entirety.

### SUBMISSION OF SEQUENCE LISTING AS ASCII TEXT FILE

This application includes an electronically submitted sequence listing in XML format. The XML file contains a sequence listing entitled "P23-0148US Sequence Listing.xml" which was created on Jul. 14, 2023 and is 17,193 bytes in size. The sequence listing contained in this XML file is part of the specification and is hereby incorporated by reference herein in its entirety.

### BACKGROUND OF THE INVENTION

### 1. FIELD OF THE INVENTION

The present invention relates to biological detection chips and application thereof, especially to biological detection chips having a plurality of transistors in parallel and to a detection method using the biological detection chips.

### 2. DESCRIPTION OF THE PRIOR ART

Biological detection techniques often rely on specific interactions between biomolecules, such as antigen-antibody binding, nucleic acid hybridization, etc., resulting in physical or chemical changes to be detected to determine the presence of biological targets in test samples. However, conventional methods of biological detection, such as immunoassays and reverse transcription-polymerase chain reaction (RT-PCR), are often difficult to perform outside of laboratory settings. These methods are complex, time-consuming, and require specialized equipment and expertise. For example, in the case of conventional detection processes for pig-related viruses, samples must be collected from pig farms and transported to a laboratory for testing. In regions with limited testing facilities, such as some countries in Southeast Asia, the waiting time for testing results may exceed one week, posing risks of delayed disease control measures.

In view of the above, conventional methods of biological detection have many drawbacks, including low sensitivity, long detection times, and complex operations. There is a need to improve biological detection methods.

### SUMMARY OF THE INVENTION

In one aspect, the present invention provides a biological detection chip, comprising a plurality of transistors disposed in an array having a plurality of rows and a plurality of columns, each of the plurality of transistors being disposed within one of the plurality of rows and one of the plurality of columns, and each of the plurality of transistors comprising:
a substrate layer comprising a shared source, a shared drain, and a channel area disposed between the shared source and the shared drain;
a floating gate disposed on the channel area and comprising a polysilicon oxide layer;
an extending metal connector disposed on the floating gate;
an extending gate disposed on the extending metal connector and electrically connected to the floating gate through the extending metal connector; and
a biological detection layer disposed on the extending gate and comprising an immobilization region and at least one biological probe immobilized on the immobilization region.

Preferably, the plurality of transistors disposed in the same row are connected in parallel. Among the plurality of transistors in the same column, the respective floating gates of adjacent transistors are disposed close to each other at approximately the same distances. The respective floating gates of adjacent transistors are positioned in close proximity to the shared source or shared drain region between the two transistors, that is the floating gates of two adjacent transistors disposed in the same column have approximately the same distances to the shared source or the shared drain between the two adjacent transistors. Alternatively, the respective floating gates of adj acent transistors partially overlap at least one part of the shared source or shared drain region between the two transistors, that is the floating gates of the two adjacent transistors disposed in the same column have approximately the same distances overlapping the shared source or the shared drain between the two adjacent transistors.

Preferably, the plurality of the biological detection layers forms a plurality of biological detection areas in parallel on the surface of the biological detection chip.

Preferably, the plurality of biological detection areas may include a micro-flow channel or a sample tank.

Preferably, the immobilization region is generated through a surface modification process on the surface of the extending gate, wherein the surface modification process is selected from the group consisting of 3-aminopropyltriethoxysilane-Glutaraldehyde (APTES-GA), 3-aminopropyltriethoxysilane-N-hydroxysuccinimide (APTES-NHS), 3-aminopropyltriethoxysilane-Biotin (APTES-Biotin), and 3-glycidoxypropyltrimethoxysilane (GPTMS) surface modification method.

Preferably, the immobilization region comprises a plurality of chemical structure selected from the group consisting of 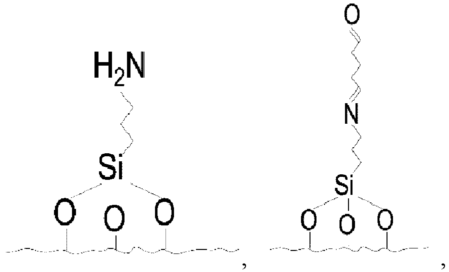 and

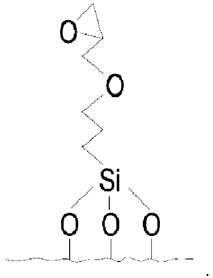

Preferably, the biological probe comprises at least one of deoxyribonucleic acid, ribonucleic acid, antibody, and aptamer. The biological probe is immobilized on the immobilization region through biological conjugation.

Preferably, the biological detection chip is for detecting porcine reproductive and respiratory syndrome virus (PRRSV), wherein the biological probe comprises at least one of synthetic oligonucleotides of SEQ ID NO: 1 to SEQ ID NO: 11.

Preferably, the biological detection chip is for detecting porcine epidemic diarrhea virus (PEDV), wherein the biological probe comprises at least one of synthetic oligonucleotides of SEQ ID NO: 12 to SEQ ID NO: 15.

Preferably, the biological detection chip is for detecting cortisol, wherein the biological probe is an anti-cortisol antibody.

In another aspect, the present invention provides a method for detecting a biological target, comprising the following steps of:
providing a biological detection chip of the present invention;
placing a blank sample in the plurality of biological detection areas connected in parallel, and measuring a gate voltage and a drain current of the plurality of transistors to establish a standard line;
placing a test sample in the plurality of biological detection areas for a predetermined time; washing the plurality of biological detection areas by the blank sample;
measuring the gate voltage and the drain current of the plurality of transistors to establish a measurement line and comparing the measurement line with the standard line to determine whether the test sample contains the biological target.

Preferably, the biological target is porcine reproductive and respiratory syndrome virus (PRRSV), porcine epidemic diarrhea virus (PEDV), or cortisol.

In another aspect, the present invention provides a method for detecting porcine reproductive and respiratory syndrome virus (PRRSV) using the aforementioned biological detection chip. The method involves using the biological detection chip to perform PRRSV detection on a test sample. The biological probe comprises at least one of synthetic oligonucleotides of SEQ ID NO: 1 to SEQ ID NO: 11. Additionally, the biological probe can also be an antibody against porcine reproductive and respiratory syndrome virus (PRRSV).

In another aspect, the present invention provides a method for detecting porcine epidemic diarrhea virus (PEDV) using the aforementioned biological detection chip. The method involves using the biological detection chip to perform PEDV detection on a test sample. The biological probe comprises at least one of synthetic oligonucleotides of SEQ ID NO: 12 to SEQ ID NO: 15. Additionally, the biological probe can also be an antibody against porcine epidemic diarrhea virus (PEDV).

In another aspect, the present invention provides a method for detecting cortisol using the aforementioned biological detection chip. The method involves using the biological detection chip to perform cortisol detection on a test sample. The biological probe is an anti-cortisol antibody.

The biological detection chip provided by the present invention offers several advantages, including high sensitivity, rapid detection, easy operation, and excellent accuracy. The integration of a biological detection layer with transistor structures enhances the sensitivity and convenience of biological detection. The detection function is improved, and the detection time is shortened through signal analysis. Additionally, the extension of the gate electrode prevents the components from being exposed to the external environment, thereby increasing the stability and service life of the biological detection chip. Moreover, by replacing different biological probes, the chip can detect various biological targets, enhancing the versatility of the biological detection chip and facilitating its implementation in various industries.

### BRIEF DESCRIPTION OF THE DRAWINGS

The accompanying drawings illustrate one or more embodiments of the invention and, together with the written description, serve to explain the principles of the invention. Wherever possible, the same reference numbers are used throughout the drawings to refer to the same or like elements of an embodiment.
FIG. 1 is a schematic diagram of the biological detection chip in accordance with one embodiment of the present disclosure.
FIG. 2A is a cross-sectional view of the biological detection chip along the dotted line AA' in FIG. 1 in one embodiment of the present disclosure.
FIG. 2B is a cross-sectional view of the biological detection chip along the dotted line AA' in FIG. 1 in another embodiment of the present disclosure.
FIG. 3A is a schematic diagram of the 3-aminopropyltriethoxysilane-Glutaraldehyde (APTES-GA) modification method in accordance with one embodiment of the present disclosure.
FIG. 3B is a schematic diagram of the 3-aminopropyltriethoxysilane-N-hydroxysuccinimide (APTES-NHS) modification method in accordance with another embodiment of the present disclosure.
FIG. 3C is a schematic diagram of the 3-aminopropyltriethoxysilane-Biotin (APTES-Biotin) modification method in accordance with another embodiment of the present disclosure.
FIG. 4 is a schematic diagram of the 3-glycidoxypropyltrimethoxysilane (GPTMS) modification method in accordance with another embodiment of the present disclosure.
FIG. 5 is a flow chart of the biological detection method in accordance with one embodiment of the present disclosure.
FIG. 6 is a circuit diagram of the biological detection method in accordance with one embodiment of the present disclosure.
FIG. 7A is an electrical curve diagram of the porcine reproductive and respiratory syndrome virus (PRRSV) detected by the biological detection chip with an antibody probe in accordance with Example 1 of the present disclosure.
FIG. 7B is a signal quantization diagram of the porcine reproductive and respiratory syndrome virus (PRRSV) detected by the biological detection chip with an antibody probe in accordance with Example 1 of the present disclosure.
FIG. 7C is an electrical curve diagram of the porcine reproductive and respiratory syndrome virus (PRRSV) detected by the biological detection chip with a DNA probe in accordance with Example 1 of the present disclosure.
FIG. 7D is a signal quantization diagram of the porcine reproductive and respiratory syndrome virus (PRRSV) detected by the biological detection chip with a DNA probe in accordance with Example 1 of the present disclosure.
FIG. 8A is a signal quantization diagram of the specificity test for the detection of porcine epidemic diarrhea virus (PEDV) by the biological detection chip in accordance with Example 2 of the present disclosure.
FIG. 8B is a signal quantization diagram of the background signal test for the detection of porcine epidemic diarrhea virus (PEDV) by the biological detection chip in accordance with Example 2 of the present disclosure.
FIG. 8C is a signal quantization diagram of the sensitivity test for the detection of porcine epidemic diarrhea virus (PEDV) by the biological detection chip in accordance with Example 2 of the present disclosure.
FIG. 9A is an electrical curve diagram of the cortisol detected by the biological detection chip in accordance with Example 3 of the present disclosure.
FIG. 9B is a signal quantization diagram of the cortisol detected by the biological detection chip in accordance with Example 3 of the present disclosure.

### DETAILED DESCRIPTION OF THE PREFERRED EMBODIMENT

In view of the drawbacks of traditional technology for biological detection, this invention incorporates chips into the biological detection technology to optimize biological detection. The biological chips of the present invention interact with target substances through recognition elements, resulting in energy changes. These energy changes are then converted into signals for subsequent detection and analysis. The biological chip offers advantages such as rapid detection, convenient operation, and high sensitivity.

It should be understood that the above general description and the detailed description below are both exemplary and explanatory, and are not intended to limit the present invention. Certain details of one or more embodiments of the present invention are described in the following disclosure. Other features or advantages of the present invention will be apparent from the non-exhaustive list of representative embodiments below and from the accompanying claims.

Unless otherwise defined herein, scientific and technical terms used in connection with the present disclosure shall have the meanings that are commonly understood by those of ordinary skill in the art. Further, unless otherwise required by context, singular terms shall include pluralities and plural terms shall include the singular. The methods and techniques of the present disclosure are generally performed according to conventional methods well-known in the art. Generally, nomenclatures used in connection with, and techniques of biochemistry, enzymology, molecular and cellular biology, microbiology, genetics and protein and nucleic acid chemistry and hybridization described herein are those well-known and commonly used in the art. The methods and techniques of the present disclosure are generally performed according to conventional methods well known in the art and as described in various general and more specific references that are cited and discussed throughout the present specification unless otherwise indicated.

As used herein, the singular form "a", "an", and "the" includes plural references unless indicated otherwise. For example, "an" excipient includes one or more excipients.

The phrase "comprising" as used herein is open-ended, indicating that such embodiments may include additional elements. In contrast, the phrase "consisting of' is closed, indicating that such embodiments do not include additional elements (except for trace impurities). The phrase "consisting essentially of' is partially closed, indicating that such embodiments may further comprise elements that do not materially change the basic characteristics of such embodiments.

As used interchangeably herein, the terms "polynucleotide" and "oligonucleotide" include single-stranded DNA (ssDNA), double-stranded DNA (dsDNA), single-stranded RNA (ssRNA) and double-stranded RNA (dsRNA), modified oligonucleotides and oligonucleosides or combinations thereof. The oligonucleotide can be linearly or circularly configured, or the oligonucleotide can contain both linear and circular segments. Oligonucleotides are polymers of nucleosides joined, generally, through phosphodiester linkages, although alternate linkages, such as phosphorothioate esters may also be used in oligonucleotides. A nucleoside consists of a purine (adenine (A) or guanine (G) or derivative thereof) or pyrimidine (thymine (T), cytosine (C) or uracil (U), or derivative thereof) base bonded to a sugar. The four nucleoside units (or bases) in DNA are called deoxyadenosine, deoxyguanosine, thymidine, and deoxycytidine. A nucleotide is a phosphate ester of a nucleoside.

As used interchangeably herein, "around", "about" and "approximately" shall generally mean plus or minus 10% of the numerical value of the number with which it is being used. Therefore, about 1% means in the range of 0.9% to 1.1 %. Numerical quantities given herein are approximate, meaning that the term "around", "about" or "approximately" can be inferred if not expressly stated.

As used herein, the term "approximately the same distances" refers to that two distances are equal, or the second distance is plus or minus 10% of the first distance. For example, the first distance is 1 centimeter, and the second distance is 1 cm or 0.9 to 1.1 cm.

As used herein, the term "APTES-GA modification method" refers to a surface modification process that involves the treatment of 3-aminopropyltriethoxysilane (APTES) followed by the treatment of glutaraldehyde (GA). For detailed information on the APTES-GA modification method, please refer to the following description of Section B "Surface Modification Process."

As used herein, the term "APTES-NHS modification method" refers to a surface modification process that involves the modification of a chip surface using 3-aminopropyltriethoxysilane (APTES), together with the treatment of the biological probe with N-hydroxysuccinimide (NHS) then conjugate the biological probe to the chip surface through the biological conjugation. For detailed information on the APTES-NHS modification method, please refer to the following description of Section B "Surface Modification Process."

As used herein, the term "APTES-Biotin modification method" refers to a surface modification process that involves the modification of a chip surface using 3-aminopropyltriethoxysilane (APTES), together with the treatment of the biological probe with biotin then conjugate the biological probe to the chip surface through the biological conjugation. For detailed information on the APTES-Biotin modification method, please refer to the following description of Section B "Surface Modification Process."

As used herein, the term "GPTMS modification method" refers to a surface modification process that involves the treatment of 3-glycidoxypropyltrimethoxysilane (GPTMS). For detailed information on the GPTMS modification method, please refer to the following description of Section B "Surface Modification Process."

As used herein, the term "biological target" refers to an object to be detected by the biological detection chip of the present invention. The biological target includes, but not limited to, a pathogen, such as porcine reproductive and respiratory syndrome virus (PRRSV) and porcine epidemic diarrhea virus (PEDV), or a biomolecule, such as cortisol.

In order to facilitate the understanding of the technical features, the contents, and the advantages of the present disclosure, and the effectiveness thereof that can be achieved, the present disclosure will be illustrated in detail below through embodiments with reference to the accompanying drawings. On the other hand, the diagrams used herein are merely intended to be schematic and auxiliary to the specification but are not necessary to be true scale and precise configuration after implementing the present disclosure. Thus, it should not be interpreted in accordance with the scale and the configuration of the accompanying drawings to limit the scope of the present disclosure on the practical implementation.

### A. Structure of Biological Detection Chip

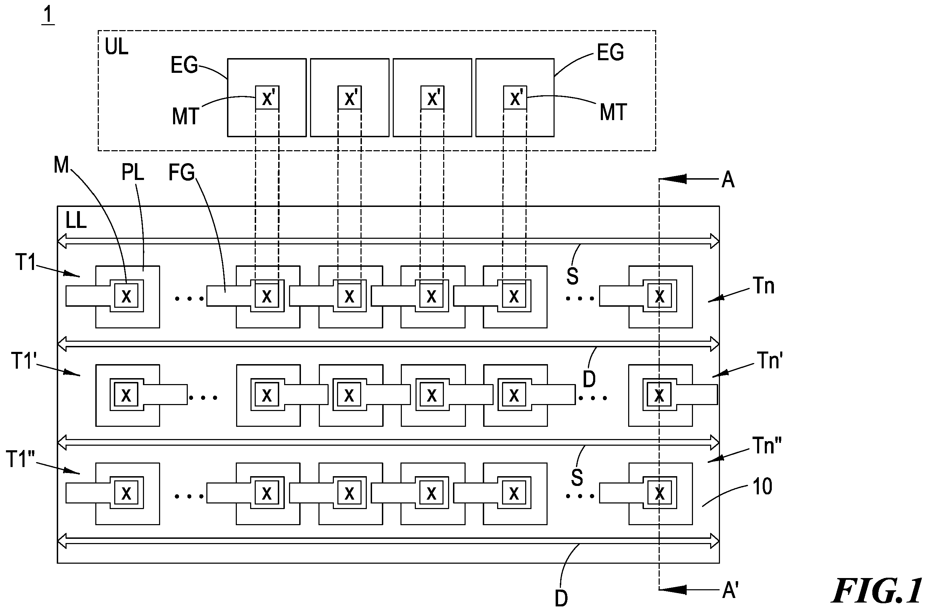

Please refer to FIG. 1, which is the schematic diagram of the biological detection chip in accordance with one embodiment of the present disclosure. As shown in FIG. 1, the biological detection chip 1 includes n transistors T1-Tn arranged in a row of an array. The array has a plurality of rows and a plurality of columns. Each transistor is disposed within one of the rows and one of the columns. The transistors in the same row are connected in parallel. The quantities of the transistors can be determined by the demand of biological detection chip 1. The structure of the transistors T1-Tn connected in parallel includes an upper layer UL and a lower layer LL. Take the transistor T1 as an example; the substrate layer 10 contains a shared source S, a shared drain D, and a channel area disposed between the shared source S and the shared drain D. The floating gate FG is disposed on the channel area between the shared source S and the shared drain D. A polysilicon oxide layer PL is disposed under the floating gate FG. An extending metal connector MT is disposed on the floating gate FG at the location of the X block and extends through the floating gate FG to the extending gate EG at the locations of the X' block. The extending gate EG is disposed on the extending metal connector MT. Therefore, the extending gate EG is electrically connected to the extending gate EG of the upper layer UL and the floating gate FG of the lower layer LL.

In addition, as shown in FIG. 1, the floating gate FG of the transistor T1 and the floating gate FG of the adjacent transistor T 1' in the lower row can be respectively close to the same shared drain D at an approximately the same distance, and the floating gate FG of the adjacent transistor T1' in the lower row and the floating gate FG of the adjacent transistors T1" below the lower row can be respectively close to the same shared source S at an approximately the same distance. That is, transistors T1, T1', and T1" are disposed in the same column, and the floating gate FG of transistor T1 and the floating gate FG of transistor T1' have approximately the same distances to the shared drain D between transistors T1 and T1'. Similarly, the floating gate FG of transistor T1' and the floating gate FG of transistor T1" have approximately the same distances to the shared source S between transistors T1' and T1".

In the present embodiment, the extending gate EG is a metal plate electrode. The extending gate EG connects to the covered polysilicon oxide layer PL through the extending metal connector MT, so that the extending gate EG of the upper layer UL may electrically connect to the floating gate FG. In other embodiments, the extending gate EG may be a metal electrode in other shapes. For example, the extending gate EG may be a spiral shape electrode. In addition, several rows of the n transistors T1-Tn connected in parallel can be arranged to form a structure with a plurality of detection layers. Between each detection layer, the adjacent detection layers share the shared source S or the shared drain D. When measuring the transistors T1-Tn, the transistors T1-Tn are connected in parallel. The shared source S may connect to the ground and the shared drain D may connect to a current measuring terminal. Based on the voltage applied to the extending gate EG, a sum of the drain current can be measured as detection data for judging the result of the biological detection.

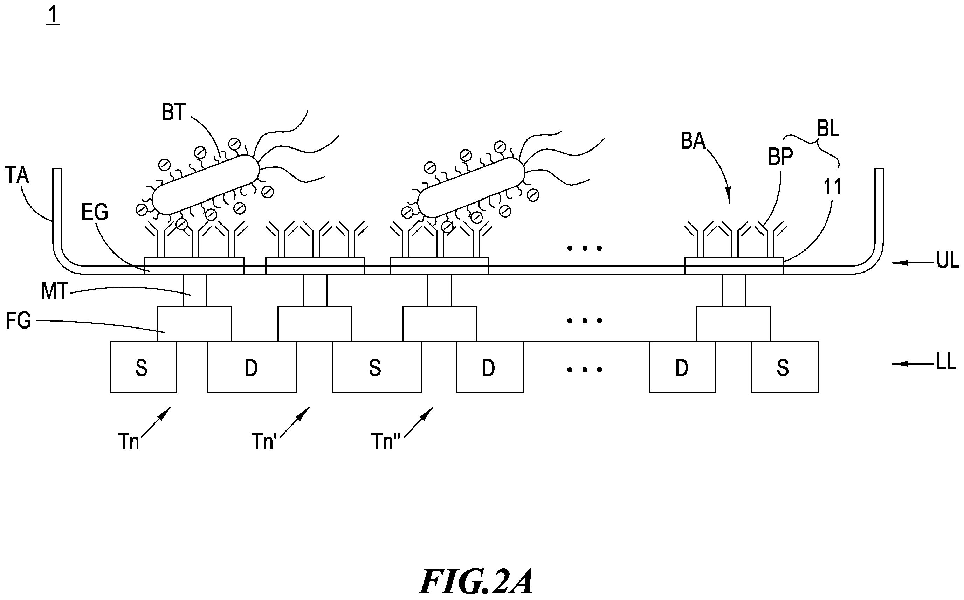

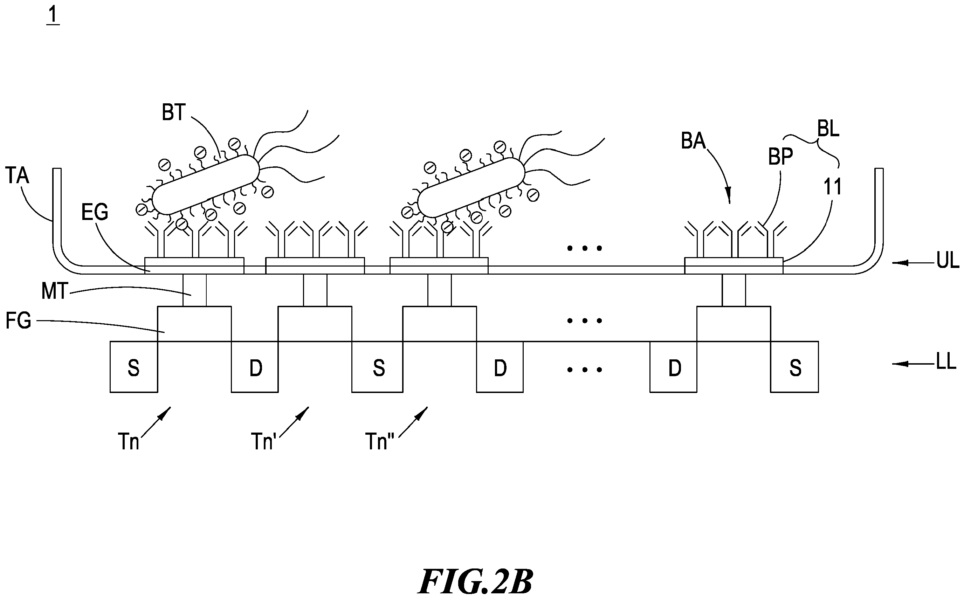

In order to conduct the biological detection, a biological detection layer BL is disposed on the extending gate EG of the transistors T1-Tn. The biological detection layer BL can bind a specific biological target BT. When a biological detection layer BL binds a specific biological target BT, the electronic feature of the transistor changes. The measurement of the current is used to determine whether there is a specific biological target BT. Regarding the biological detection layer BL, FIGs. 2A and 2B show two embodiments of the cross-sectional views of the biological detection chip along the dotted line AA' in FIG. 1. As shown in FIGs. 2A and 2B, the biological detection chip 1 includes a plurality of detection layers. Each of the plurality of detection layers includes the upper layer UL and the lower layer LL, and the biological detection layer BL is disposed on the upper layer UL.

As shown in FIGs. 2A and 2B, take the transistor Tn as an example; the transistor Tn includes the shared source S and the shared drain D. The floating gate FG is disposed on the channel area between the shared source S and the shared drain D. The floating gate FG is electrically connected to the extending gate EG through the extending metal connector MT. As shown in FIG. 2A, in one embodiment, each of the floating gate FG of the transistor Tn and the floating gate FG of the adjacent transistor Tn' may cover at least a part of the same shared drain D; each of the floating gate FG of the adjacent transistor Tn' and the floating gate FG of another transistor Tn" which abuts upon the adjacent transistor Tn' may cover at least a part of the same shared source S. In a preferable embodiment, the transistors Tn, Tn', and Tn" are disposed in the same column, and the floating gate FG of transistor Tn and the floating gate FG of transistor Tn' have approximately the same distances overlapping the shared drain D between transistors Tn and Tn'. Similarly, the floating gate FG of transistor Tn' and the floating gate FG of transistor Tn" have approximately the same distances overlapping the shared source S between transistors Tn' and Tn". As shown in FIG. 2B, in another embodiment, each of the floating gate FG of the transistor Tn and the floating gate FG of the adjacent transistor Tn' may be positioned in close proximity to the same shared drain D; each of the floating gate FG of transistor Tn' and the floating gate FG of the other adjacent transistor Tn" may be positioned in close proximity to the same shared source S. In a preferable embodiment, the transistors Tn, Tn', and Tn" are disposed in the same column, and the floating gate FG of transistor Tn and the floating gate FG of transistor Tn' have approximately the same distances to the shared drain D between transistors Tn and Tn'. Similarly, the floating gate FG of transistor Tn' and the floating gate FG of transistor Tn" have approximately the same distances to the shared source S between transistors Tn' and Tn".

The biological detection layer BL is disposed on the extending gate EG. The immobilization region 11 is generated on the extending gate EG surface through a surface modification process. The plurality of biological probes BP may be immobilized on the immobilization region 11.

The biological probes BP used herein may be biomolecules, including, but not limited to, deoxyribonucleic acid (DNA), ribonucleic acid (RNA), antibody, and aptamer. The biological probes BP are immobilized on the immobilization region 11 through biological conjugation. When biological detection is conducted, the biological probes BP bind the biological targets BT in a sample, and the generated charge may affect the drain current signal detected from the transistor Tn. Based on the drain current signal, the biological target BT corresponding to the specific biological probes BP or the quantities of the biological target BT can be determined accordingly.

The biological detection chip 1 may form a detection layer by several transistors connected in parallel. The plurality of detection layers may further form a detection array, so as to form a plurality of biological detection areas BAs on the surface of the biological detection chip 1. A sample tank TA may be set at the biological detection layer BL to create an accommodating space, so as to contain biological detection solution in the biological detection areas BAs. Therefore, the biological target BT in the biological detection solution can be accommodated within the biological detection areas BAs. The biological detection solution may cover the surface of the biological detection chip 1, so that the biological target BT and the biological probes BP of the biological detection layer BL can contact and bind to each other. In the present embodiment, the accommodating space is a sample tank TA. However, the accommodating space may be in a different form, such as, but not limited to, a microflow channel disposed at the biological detection layer BL.

In summary, the present invention provides a biological detection chip, comprising a plurality of transistors disposed in an array having a plurality of rows and a plurality of columns, each of the plurality of transistors being disposed within one of the plurality of rows and one of the plurality of columns, and each of the plurality of transistors comprising:
a substrate layer comprising a shared source, a shared drain, and a channel area disposed between the shared source and the shared drain;
a floating gate disposed on the channel area and comprising a polysilicon oxide layer;
an extending metal connector disposed on the floating gate;
an extending gate disposed on the extending metal connector and electrically connected to the floating gate through the extending metal connector; and
a biological detection layer disposed on the extending gate and comprising an immobilization region and at least one biological probe immobilized on the immobilization region.

Preferably, the plurality of transistors disposed in the same row are connected in parallel. Among the plurality of transistors in the same column, the respective floating gates of adjacent transistors are disposed close to each other at approximately equal distances. The floating gates of two adjacent transistors disposed in the same column have approximately the same distances to the shared source or the shared drain between the two adjacent transistors. Alternatively, the floating gates of the two adjacent transistors disposed in the same column have approximately the same distances overlapping the shared source or the shared drain between the two adjacent transistors.

Preferably, a plurality of the biological detection layers forms a plurality of biological detection areas in parallel on the surface of the biological detection chip.

Preferably, the immobilization region comprises a plurality of chemical structure selected from the group consisting of 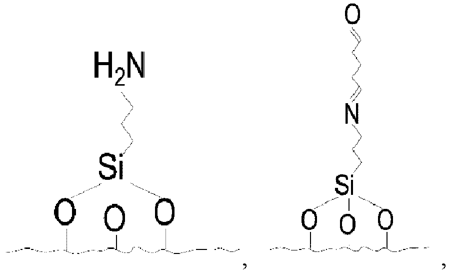 and

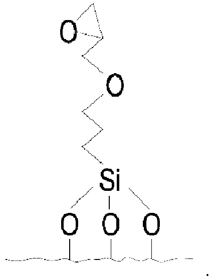

### B. Surface Modification Process

The immobilization region of the biological detection chip of the present disclosure is generated through a surface modification process on the surface of the extending gate. Preferably, the surface modification process is one of APTES-GA modification method, APTES-NHS modification method, APTES-Biotin modification method, and GPTMS modification method. Detailed information on each of the surface modification process is described below.

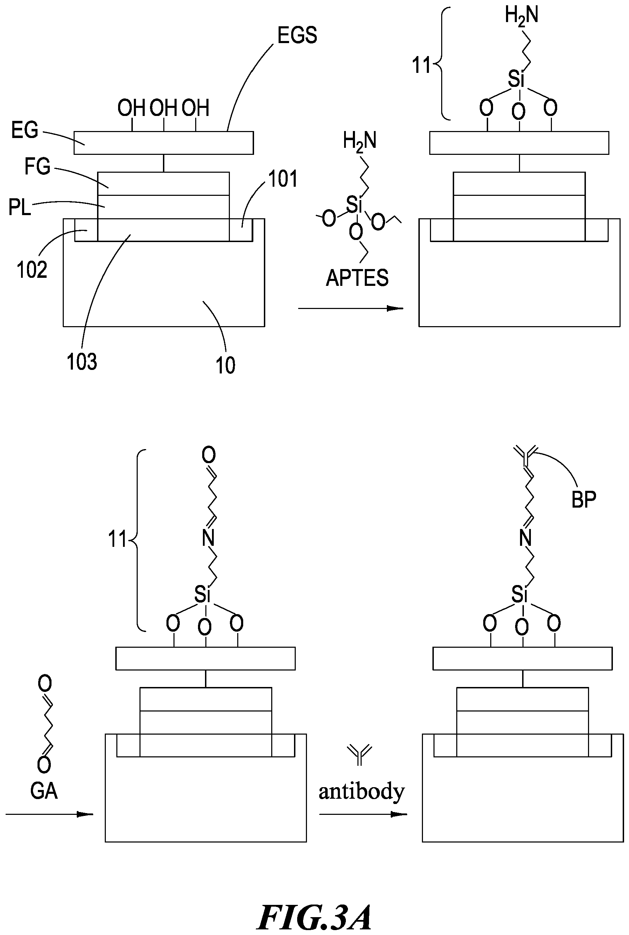

As shown in FIGs. 3A to 4, the production process of the biological detection chip firstly forms the source area 101, the drain area 102 and the channel area 103. Then the floating gate FG is formed on the channel area 103. The polysilicon oxide layer PL is disposed under the floating gate FG, and the metal connector is disposed on the floating gate FG for further connecting to the extending gate EG.

As shown in FIG. 3A, the steps involved in the APTES-GA modification method are described as follows:
A packaged chip was first sonicated in acetone and then sonicated in 95% ethanol, followed by drying at 90-120°C to remove ethanol. The chip was then subjected to oxygen plasma treatment to incorporate hydroxyl groups (-OH) onto the surface of the extending gate EGS. After that, the chip was immersed in 3-aminopropyltriethoxysilane (APTES) dissolved in ethanol for approximately 30 minutes, followed by sonication. Then, the chip was dried at 90-120°C to remove ethanol and make amine groups (-NH₂) of APTES bind to the hydroxylated surface of the extending gate EGS.
Then, the chip was immersed in glutaraldehyde (GA) solution for approximately 30 minutes, so that the surface of the extending gate EGS was modified with aldehyde groups (-CHO). Subsequently, multiple biological probes BP with amino groups (-NH₂) were conjugated to the aldehyde groups in the immobilization region 11, so the biological probes BP were immobilized on the chip.
After treatment with a blocking agent, such as bovine serum albumin (BSA), the chip was washed, dried with nitrogen gas, and placed in a mold for vacuum storage or further use.

After surface modification by APTES-GA modification method described above, the immobilization region contains a plurality of chemical structure as shown below:

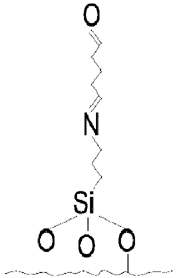

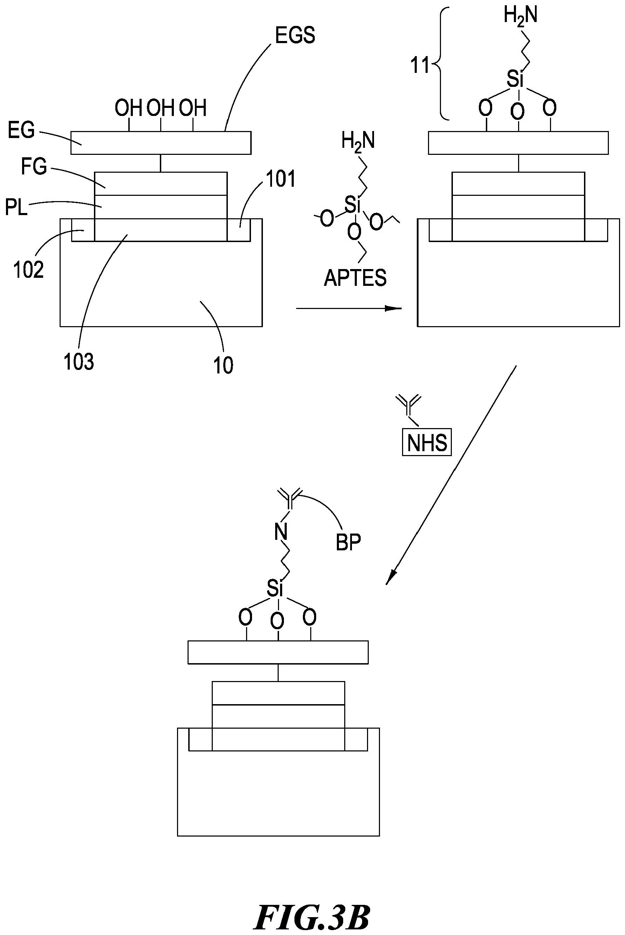

As shown in FIG. 3B, the steps involved in the APTES-NHS modification method are described as follows:
A packaged chip was first sonicated in acetone and then sonicated in 95% ethanol, followed by drying at 90-120°C to remove ethanol. The chip was then subjected to oxygen plasma treatment to incorporate hydroxyl groups (-OH) onto the surface of the extending gate EGS. After that, the chip was immersed in 3-aminopropyltriethoxysilane (APTES) dissolved in ethanol for approximately 30 minutes, followed by sonication. Then, the chip was dried at 90-120°C to remove ethanol and make amine groups (-NH₂) of APTES bind to the hydroxylated surface of the extending gate EGS.
Subsequently, multiple biological probes BP with N-hydroxysuccinimide (NHS) groups were conjugated to the amine groups in the immobilization region 11 for approximately 30 minutes, so the biological probes BP were immobilized on the chip.
After treatment with a blocking agent, such as BSA, the chip was washed, dried with nitrogen gas, and placed in a mold for vacuum storage or further use.

After surface modification by APTES-NHS modification method described above, the immobilization region contains a plurality of chemical structure as shown below:

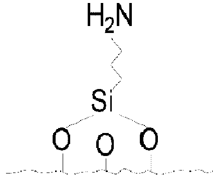

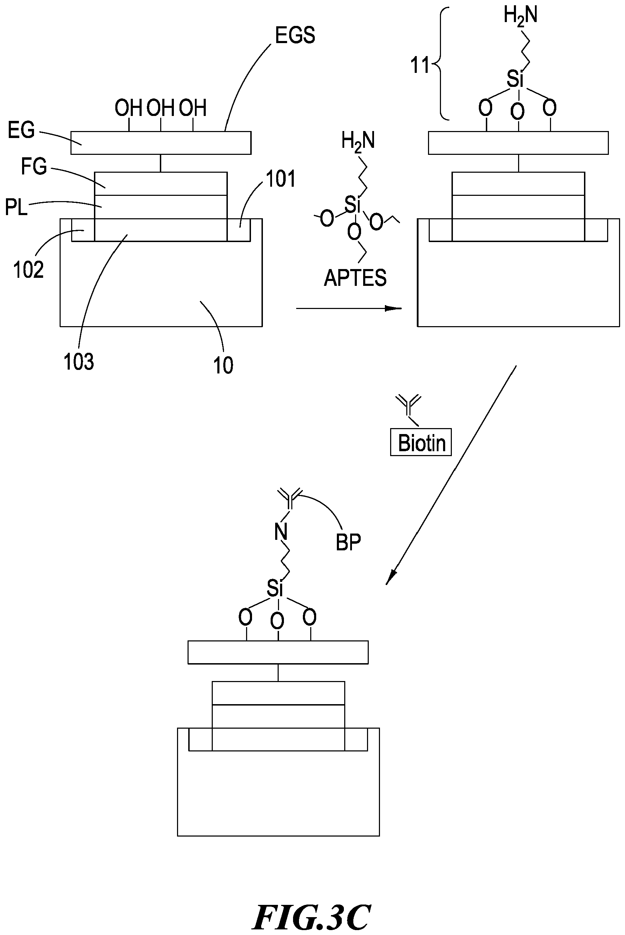

As shown in FIG. 3C, the steps involved in the APTES-Biotin modification method are described as follows:
A packaged chip was first sonicated in acetone and then sonicated in 95% ethanol, followed by drying at 90-120°C to remove ethanol. The chip was then subjected to oxygen plasma treatment to incorporate hydroxyl groups (-OH) onto the surface of the extending gate EGS. After that, the chip was immersed in 3-aminopropyltriethoxysilane (APTES) dissolved in ethanol for approximately 30 minutes, followed by sonication. Then, the chip was dried at 90-120°C to remove ethanol and make amine groups (-NH₂) of APTES bind to the hydroxylated surface of the extending gate EGS.
Subsequently, multiple biological probes BP with biotin groups were conjugated to the amine groups in the immobilization region 11 for approximately 30 minutes, so the biological probes BP were immobilized on the chip.
After treatment with a blocking agent, such as BSA, the chip was washed, dried with nitrogen gas, and placed in a mold for vacuum storage or further use.

After surface modification by APTES-Biotin modification method described above, the immobilization region contains a plurality of chemical structure as shown below:

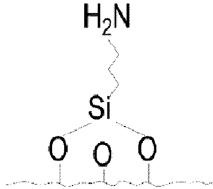

Preferably, 3-aminopropyltriethoxysilane (APTES) can be replaced with epoxy resin solution to modify the surface of the extending gate EGS on the chip.

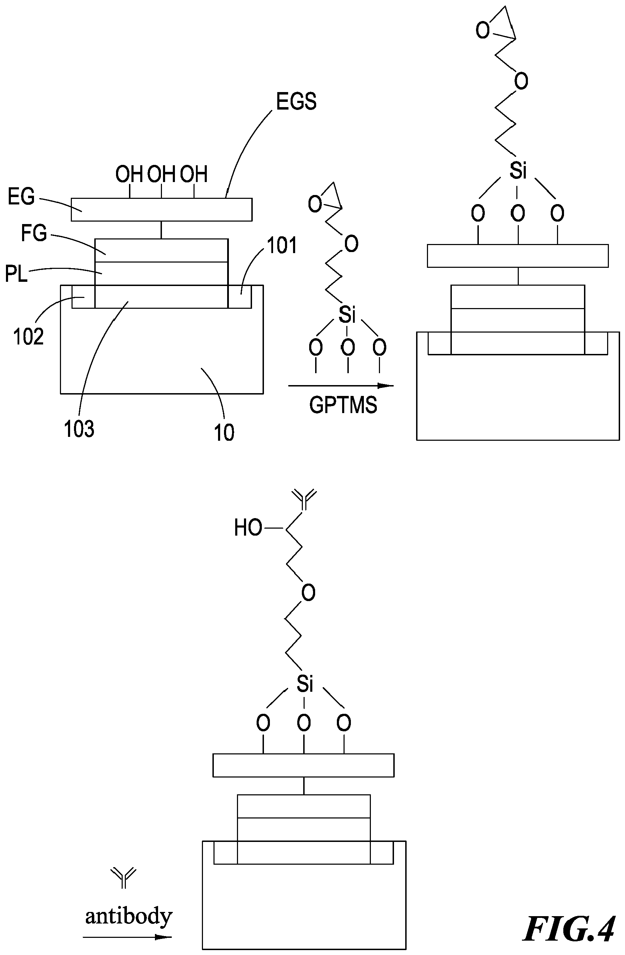

As shown in FIG. 4, the steps involved in the GPTMS modification method are described as follows:
A packaged chip was first sonicated in acetone and then sonicated in 95% ethanol, followed by drying at 90-120°C to remove ethanol. The chip was then subjected to oxygen plasma treatment to incorporate hydroxyl groups (-OH) onto the surface of the extending gate EGS. After that, the chip was immersed in 3-glycidoxypropyltrimethoxysilane (GPTMS) dissolved in absolute ethanol and then sonicated in absolute ethanol. Then, the chip was dried at 90-120°C to remove ethanol and make epoxide group of GPTMS bind to the hydroxylated surface of the extending gate EGS.
Subsequently, multiple biological probes BP with amino groups (-NH₂) were conjugated to the epoxide groups in the immobilization region 11, so the biological probes BP were immobilized on the chip.
After treatment with a blocking agent, such as BSA, the chip was washed, dried with nitrogen gas, and placed in a mold for vacuum storage or further use.

After surface modification by GPTMS modification method described above, the immobilization region contains a plurality of chemical structure as shown below:

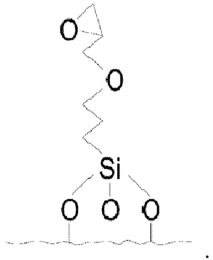

### C. Biological Detection Method

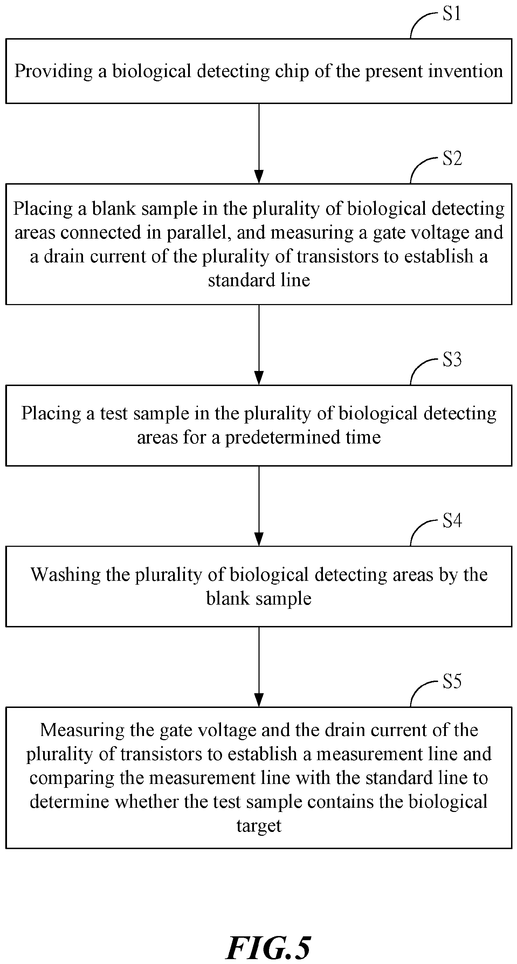

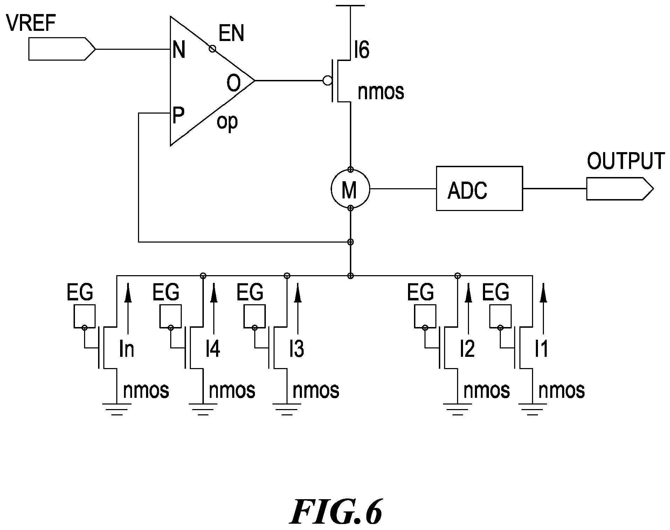

The present invention also provides a biological detection method, which includes the following steps (S1-S5) as shown in FIG. 5:
Step S1: providing a biological detection chip of the present invention.
Step S2: placing a blank sample in the plurality of biological detection areas connected in parallel, and measuring a gate voltage and a drain current of the plurality of transistors to establish a standard line. As shown in FIG. 6, which is a circuit diagram of the biological detection method in accordance with one embodiment of the present disclosure, the biological detection chip includes detection layers formed by the transistors nmos connected in parallel. The shared sources S of the transistors nmos connect to the ground, and the shared drains D connect to the current measuring device M. The gate electrode extends to the extending gate EG. After a blank sample, such as a neutral buffer, is placed in the biological detection areas to cover the extending gate EG of the biological detection layer, different gate voltages, for example, 0V to 2V, are applied to the extending gate EG. Then, the current measuring device M measures the output current I1-In of the shared drain D. Since the transistors nmos are connected in parallel, the sum of the output current I1-In can be converted to digital signal through the voltage converter ADC. The information of the drain current is output from the output terminal OUTPUT and the standard line of the measurement is established accordingly.

In the present embodiment, the biological detection chip further comprises an amplifier OP. The input terminal of the amplifier OP connects to a reference voltage VREF and the shared drain D in parallel. The output terminal of the amplifier OP is coupled to the control terminal of the transistor nmos. The transistor nmos is coupled to the current measuring device M. In other embodiments, the shared source S and the shared drain D may connect to a voltage source and signal collecting device through a contact. The information of the source current can be collected by an external device.

Step S3: placing a test sample in the plurality of biological detection areas for a predetermined time. The test sample solution is placed in the biological detection areas. For example, the test sample is placed in the sample tank for a period of time, so that the biological target can bind to the biological probes of the biological detection layer. The reaction time for the test sample is determined by the biological target species.

Step S4: washing the plurality of biological detection areas by the blank sample. Preferably, steps S3 and S4 are repeated several times to ensure that the biological targets in the test sample fully bind to the biological probes. The number of repetitions is determined by various parameters, such as the concentration of the test sample and the binding characteristics of the biological probe.

Step S5: measuring the gate voltage and the drain current of the plurality of transistors to establish a measurement line, and comparing the measurement line with the standard line to determine whether the test sample contains the biological target. After the reaction (S3) and washing (S4) steps, different gate voltages are applied to the extending gate EG again. The output current I1-In of the shared drain D are measured by the current measuring device M and the sum of the output current I1-In is converted to a digital signal through the voltage converter ADC. The information of the drain current is output from the output terminal OUTPUT, and the measurement line of the test sample is established accordingly. The measurement line is then compared to the standard line to determine how much the measurement line deviates from the standard line, and to further determine whether the test sample contains the biological target based on the level of the deviation. When a test sample does not contain the biological target, the biological probes on the detection chip do not bind to anything; there is no change of the gate charge of the chip, so the measurement line does not obviously deviate from the standard line. In contrast, when a test sample contains the biological target, the biological probes on the detection chip bind to the biological target; there is change of the gate charge of the chip, so the measurement line deviates from the standard line. Therefore, if there is no obvious deviation of a measurement line from the standard line, it is determined that the test sample does not contain the biological target. However, if the deviation of a measurement line from the standard line exceeds a predetermined level, it is determined that the test sample contains the biological target.

Preferably, the biological target is one of porcine reproductive and respiratory syndrome virus (PRRSV), porcine epidemic diarrhea virus (PEDV), and cortisol. By using different biological probes, the biological detection chip of the present invention can be used to detect different biological targets. In addition, the biological detection chip of the present invention can detect various types of test samples, such as pathogens or proteins in human saliva or blood.

Preferably, during electrical measurements, a voltage ranging from 0V to 2V (0, 0.03, 0.06...2V) or 1.5V to 3V, depending on the biological target to be detected, is applied to the sample tank. There are 63 voltage values in total. The measurement software records the voltage value transmitted from the sensing area to the semiconductor device under each applied voltage. This process generates 63 sets of numerical values, which are then plotted to produce an electrical curve. In addition, the term "chip signal value" as used herein refers to quantifying the signal. This quantification is calculated through a trapezoidal formula, which is employed to calculate the area between the standard curve and the sample curve. The calculation is performed as follows: |((Target2+Target1)^{∗}1/2)-((Baseline2+Baseline1)^{∗}1/2))| +|((Target3+Target2)^{∗}1/2)-((Baseline3+Baseline2)^{∗}1/2))|+... +|((Target63+Target62)^{∗}1/2)-((Baseline63+Baseline62)^{∗}1/2))|.

The present invention is further illustrated by the following Examples, which in no way should be construed as further limiting. The entire contents of all of the references (including literature references, issued patents, published patent applications, and co-pending patent applications) cited throughout this application are hereby expressly incorporated by reference.

### ENUMERATED EMBODIMENTS

Embodiment 1. A biological detection chip, comprising a plurality of transistors disposed in an array having a plurality of rows and a plurality of columns, each of the plurality of transistors being disposed within one of the plurality of rows and one of the plurality of columns, and each of the plurality of transistors comprising:
   a substrate layer comprising a shared source, a shared drain, and a channel area disposed between the shared source and the shared drain;
   a floating gate disposed on the channel area and comprising a polysilicon oxide layer;
   an extending metal connector disposed on the floating gate;
   an extending gate disposed on the extending metal connector and electrically connected to the floating gate through the extending metal connector; and
   a biological detection layer disposed on the extending gate and comprising an immobilization region and at least one biological probe immobilized on the immobilization region.
Embodiment 2. The biological detection chip of embodiment 1, wherein a plurality of the biological detection layers forms a plurality of biological detection areas in parallel on the surface of the biological detection chip.
Embodiment 3. The biological detection chip of embodiment 1 or 2, wherein the plurality of transistors disposed in a same row of the plurality of rows are connected in parallel.
Embodiment 4. The biological detection chip of any one of embodiments 1 to 3, wherein the floating gates of two adjacent transistors disposed in a same column of the plurality of columns have approximately the same distances to a shared source or a shared drain between the two adjacent transistors.
Embodiment 5. The biological detection chip of any one of embodiments 1 to 3, wherein the floating gates of two adjacent transistors disposed in a same column of the plurality of columns have approximately the same distances overlapping a shared source or a shared drain between the two adjacent transistors.
Embodiment 6. The biological detection chip of any one of embodiments 1 to 5, wherein the immobilization region is generated through a surface modification process on the surface of the extending gate, wherein the surface modification process is selected from the group consisting of 3-aminopropyltrimethoxysilane-Glutaraldehyde (APTES-GA) modification method, 3-aminopropyltrimethoxysilane-N-hydroxysuccinimide (APTES-NHS) modification method, 3-aminopropyltrimethoxysilane-Biotin (APTES-Biotin) modification method, and 3-glycidoxypropyltrimethoxysilane (GPTMS) modification method.
Embodiment 7. The biological detection chip of any one of embodiments 1 to 6, wherein the immobilization region comprises a plurality of chemical structure selected from the group consisting of 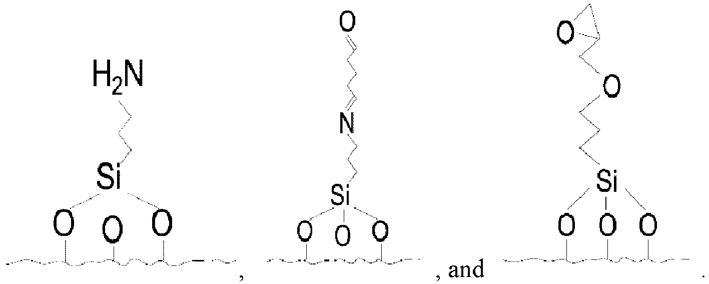
Embodiment 8. The biological detection chip of any one of embodiments 1 to 7, wherein the biological probe comprises at least one of deoxyribonucleic acid, ribonucleic acid, antibody, and aptamer.
Embodiment 9. The biological detection chip of any one of embodiments 1 to 8, wherein the plurality of biological detection areas comprises a micro-flow channel or a sample tank.
Embodiment 10. The biological detection chip of any one of embodiments 1 to 9, wherein the biological detection chip is for detecting porcine reproductive and respiratory syndrome virus (PRRSV).
Embodiment 11. The biological detection chip of embodiment 10, wherein the biological probe comprises at least one of synthetic oligonucleotides of SEQ ID NO: 1 to SEQ ID NO: 11.
Embodiment 12. The biological detection chip of any one of embodiments 1 to 9, wherein the biological detection chip is for detecting porcine epidemic diarrhea virus (PEDV).
Embodiment 13. The biological detection chip of embodiment 12, wherein the biological probe comprises at least one of synthetic oligonucleotides of SEQ ID NO: 12 to SEQ ID NO: 15.
Embodiment 14. The biological detection chip of any one of embodiments 1 to 9, wherein the biological detection chip is for detecting cortisol.
Embodiment 15. The biological detection chip of embodiment 14, wherein the biological probe is an anti-cortisol antibody.
Embodiment 16. A method for detecting a biological target, comprising the following steps of:
   providing a biological detection chip of any one of embodiments 1 to 15;
   placing a blank sample in the plurality of biological detection areas connected in parallel, and
   measuring a gate voltage and a drain current of the plurality of transistors to establish a standard line;
   placing a test sample in the plurality of biological detection areas for a predetermined time;
   washing the plurality of biological detection areas by the blank sample;
   measuring the gate voltage and the drain current of the plurality of transistors to establish a measurement line; and
   comparing the measurement line with the standard line to determine whether the test sample contains the biological target.
Embodiment 17. The biological detection method of embodiment 16, wherein the biological target is porcine reproductive and respiratory syndrome virus (PRRSV).
Embodiment 18. The biological detection method of embodiment 17, wherein the biological probe comprises at least one of synthetic oligonucleotides of SEQ ID NO: 1 to SEQ ID NO: 11.
Embodiment 19. The biological detection method of embodiment 16, wherein the biological detection chip is for detecting porcine epidemic diarrhea virus (PEDV).
Embodiment 20. The biological detection method of embodiment 19, wherein the biological probe comprises at least one of synthetic oligonucleotides of SEQ ID NO: 12 to SEQ ID NO: 15.
Embodiment 21. The biological detection method of embodiment 16, wherein the biological detection chip is for detecting cortisol
Embodiment 22. The biological detection method of embodiment 21, wherein the biological probe is an anti-cortisol antibody.

### EXAMPLES

### Example 1 Detection of Porcine Reproductive and Respiratory Syndrome Virus (PRRSV)

### Example 1.1 Detection with an Antibody Probe

In this example, the biological target is PRRSV. APTES-GA modification method was used to modify the surface of the biological detection chip in this example, and an antibody (PRRSV-N Protein antibody, Cat# orb526689, Biorbyt Ltd., Cambridge, UK) was used as the biological probe.

The test samples in this example were purified PRRSV viral protein and purified PEDV viral protein. The protein samples were prepared by sequential dilution with phosphate buffered saline (PBS), followed by cell lysis with radioimmunoprecipitation assay buffer (RIPA) for 15 minutes. The prepared protein samples were further diluted with 25 mM Tris-HCl buffer. After dilution, the concentrations of viral proteins in the test samples were as follows: 1 fg/mL PEDV (Sample 1, as the negative control), 0.1 fg/mL PRRSV (Sample 2), 100 fg/mL PRRSV (Sample 3), 100 pg/mL PRRSV (Sample 4), and 100 ng/mL PRRSV (Sample 5). These samples were prepared for examining the protein detection efficacy of the biological detection chip for PRRSV.

In some embodiments, the biological probe is an anti-PRRSV antibody, which can be a monoclonal or polyclonal antibody. Examples of anti-PRRSV antibodies include, but are not limited to, PRRSV-N Protein antibody (Cat# orb526689, Biorbyt Ltd.), Recombinant Mouse Anti-PRRSV ORF7 Antibody (ICA7) (Cat# EPAF-0626LC, Creative Biolabs Inc., Shirley, NY, USA), and PRRSV-GP5 Polyclonal Antibody (Cat# BS-4504R, Bioss Antibodies, Wobum, MA,USA).

The experimental procedure of this example includes the following steps:
add 100 µL of the blank sample (Bis-tris propane, BTP) into the sample tank, and incubate approximately 10 minutes at room temperature;
perform electrical measurements to establish a baseline (standard curve);
add 100 µL of the test sample to the sample tank, and incubate approximately 30 minutes at room temperature;
remove the test sample and wash the sample tank with blank sample;
add blank sample (BTP) into the sample tank again, and incubate approximately 10 minutes at room temperature;
perform electrical measurements to obtain electrical curves of the sample measurement results (sample curves); and
compare the standard curve and the sample curves for further analysis.

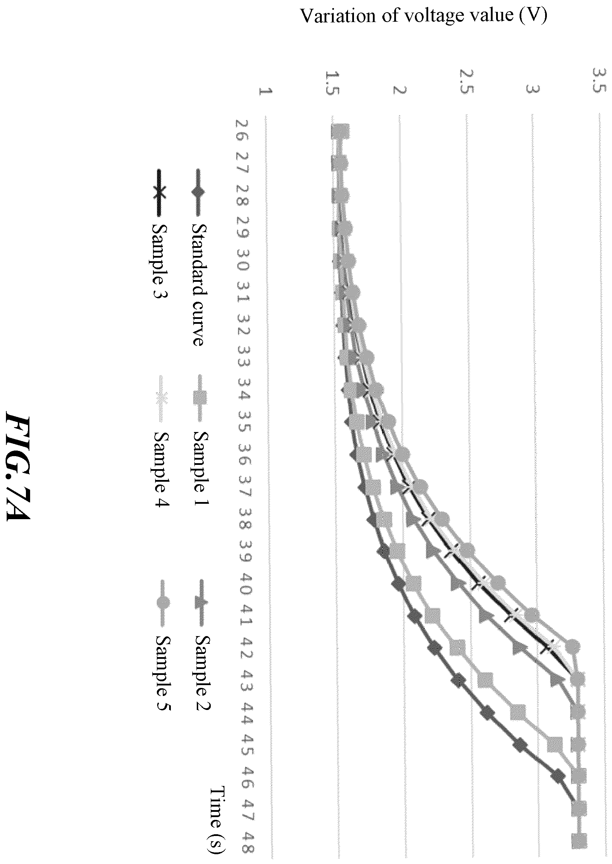

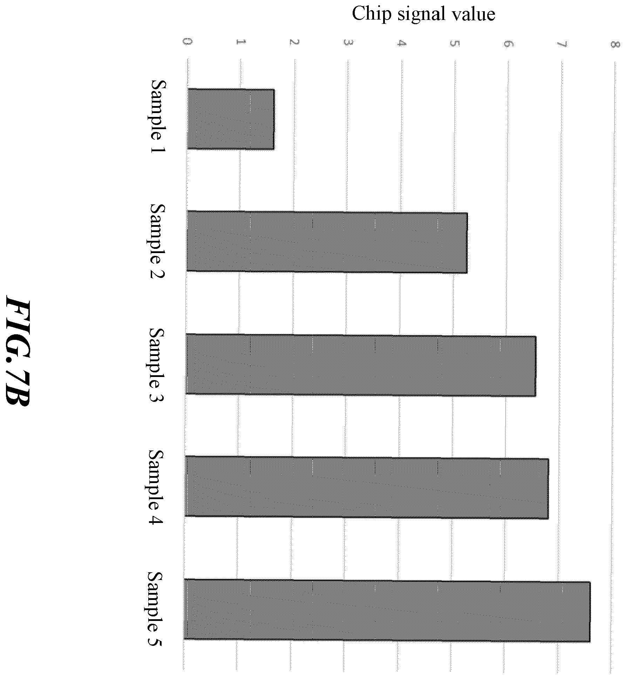

Please refer to FIGs. 7A and 7B for the experimental results. As shown in FIG. 7A, the electrical curves of Samples 2 to 5 (which are samples containing PRRSV purified viral protein) significantly deviate from the standard curve (baseline) in a concentration-dependent manner, indicating a potentially quantitative effect of the biological detection chip of the present invention. In addition, the electrical curve of the PEDV sample (Sample 1, negative control) is significantly close to the standard curve compared to the electrical curves of Samples 2 to 5, indicating that the biological detection chip of the present invention is highly specific to PRRSV. Furthermore, as shown in FIG. 7B, after quantifying the signals, the results show that the biological detection chip of the present invention detects the presence of PRRSV viral protein at an extremely low concentration of 0.1 fg/mL (Sample 2), demonstrating the high sensitivity of the chip.

### Example 1.2 Detection with Nucleic Acid Probes

In this example, the biological target is PRRSV. GPTMS modification method was used to modify the surface of the biological detection chip in this example, and a nucleic acid probe was used as the biological probe. The nucleic acid probe comprised at least one of synthetic oligonucleotides of SEQ ID NO: 1 to SEQ ID NO: 11.

A sample containing PRRSV was used in this example. The sample was subjected to cell lysis with VLL buffer (LabTurbo^{™} Virus Mini Kit, LVN480-200, LabTurbo Biotech Corporation, Taipei, Taiwan) for 15 minutes, and then diluted with 25 mM Tris-HCl buffer to a concentration of 0.24 copies/µL of PRRSV (Sample 6) and 240 copies/µL of PRRSV (Sample 7). The quality control (negative control) of this example contained only buffer solution. These samples were prepared for examining the RNA fragment detection efficacy of the biological detection chip for PRRSV. The experimental procedure was the same as described in Example 1.1.

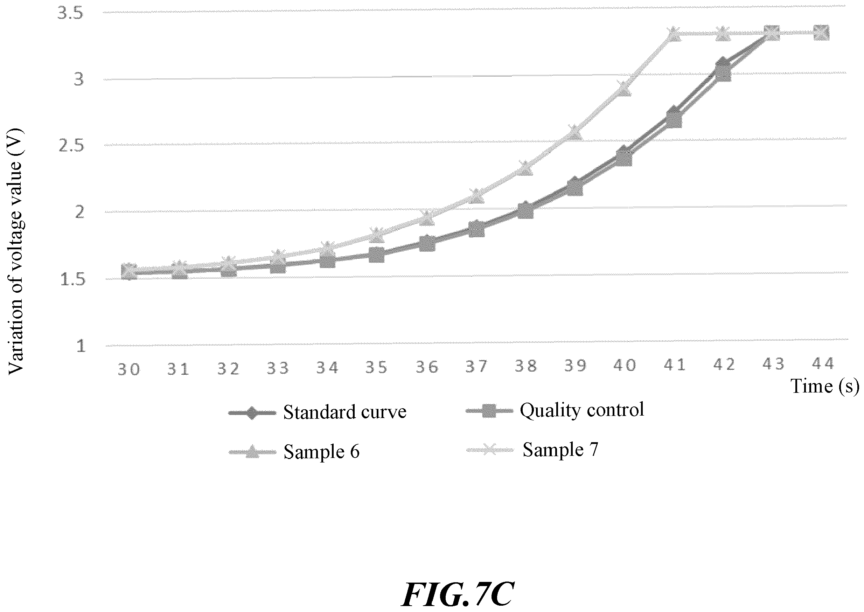

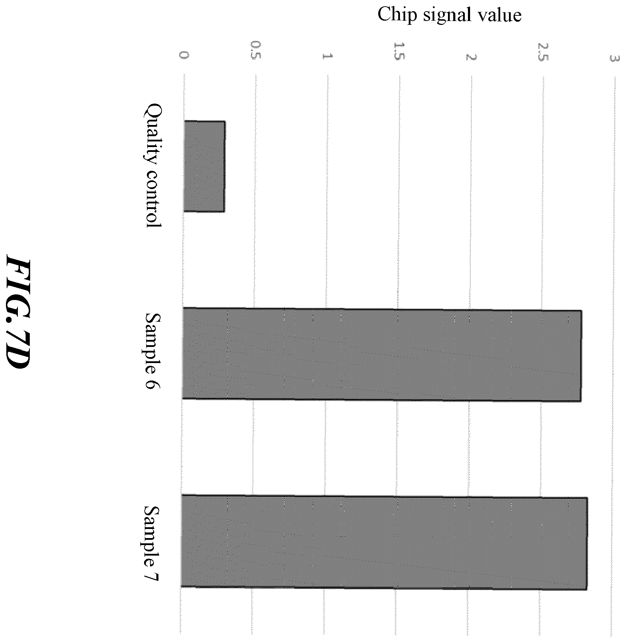

Please refer to FIGs. 7C and 7D for the experimental results. As shown in FIG. 7C, the electrical curves of Samples 6 and 7 significantly deviate from the standard curve (baseline) in a slightly concentration-dependent manner, indicating a potentially quantitative effect of the biological detection chip of the present invention. Furthermore, as shown in FIG. 7D, after quantifying the signals, the results show that the biological detection chip of the present invention detects the presence of PRRSV RNA at a concentration of at least 0.24 copies/µL in a test sample. In contrast, it requires a Ct value of above 36 to detect a positive signal of a PRRSV RNA sample of 0.24 copies/µL by traditional qPCR. These results indicate that the biological detection chip of the present invention exhibits a high sensitivity compared to conventional detection techniques.

### Example 2 Detection of Porcine Epidemic Diarrhea Virus (PEDV)

In this example, the biological target is PEDV. APTES-NHS modification method was used to modify the surface of the biological detection chip in this example, and a nucleic acid probe was used as the biological probe. The nucleic acid probe comprises at least one of synthetic oligonucleotides of SEQ ID NO: 12 to SEQ ID NO: 15.

### Example 2.1 Specificity Test with Samples Containing Different Pathogens

The test samples in this example were samples containing different pig pathogens, including *Escherichia coli* (E. coli), *Salmonella choleraesuis* (Sal. C), swine delta coronavirus (SDCoV), transmissible gastroenteritis virus (TGEV), rotavirus (RVs), and PEDV. These samples were subjected to cell lysis with VLL lysis buffer (LabTurbo^{™} Virus Mini Kit, LVN480-200) for 10 minutes to obtain the lysis solution of each sample. Then, the concentrations of the samples were adjusted by dilution with 25 mM Tris-HCl buffer. These samples were prepared for examining the detection specificity of the biological detection chip for PEDV.

The experimental procedure was the same as described in Example 1.1, except that in this example, the incubation time for the blank sample (BTP) and the test samples was 5 and 15 minutes, respectively.

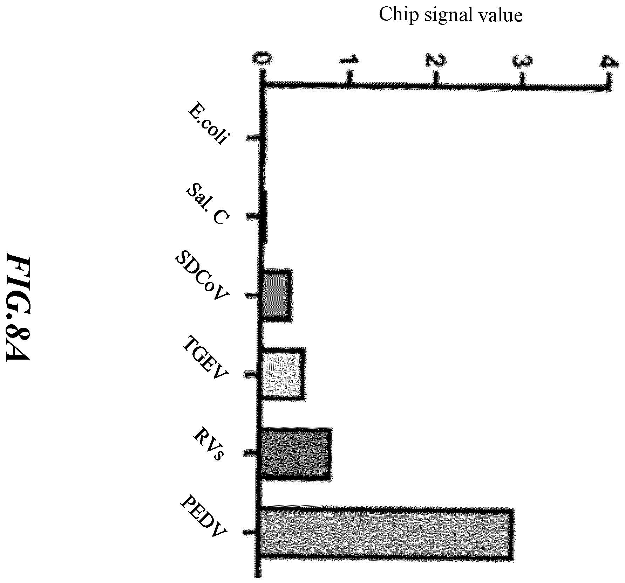

The test results are shown in FIG. 8A. The PEDV sample exhibits the strongest signal, while the signals of other lysed pathogens such as E. coli, Sal. C, SDCoV, TGEV, and RVs are all significantly lower than the PEDV sample, indicating that the biological detection chip of the present invention has high specificity to PEDV.

### Example 2.2 Test with Samples from Different Parts of a PEDV-free Pig

In this example, samples collected from different parts of a PEDV-free pig were tested to determine background signal values of these PEDV-negative samples and to further determine suitable sample types with low background signals for detecting pig pathogens with the biological detection chip of the present invention. The PEDV-negative samples used in this example included rectal swab samples (RI), ileum contents samples (CI), feces samples (FC), and small intestinal mucosal emulsion samples (SI). Lysed PEDV solution was used as positive control. The experiment was repeated six times (n=6). The experimental procedure was the same as described in Example 2.1.

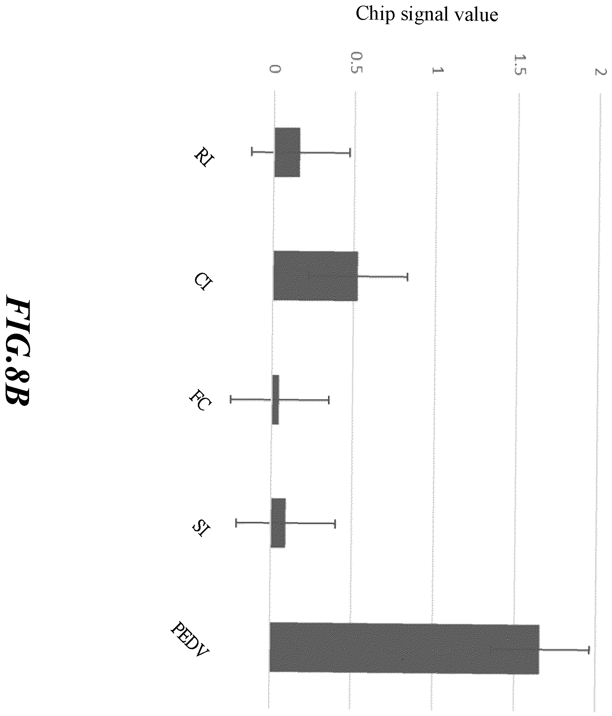

The test results are shown in FIG. 8B. The signal values of the RI samples, CI samples, FC samples, and SI samples are all significantly lower than the lysed PEDV solution samples (PEDV), indicating that the biological detection chip of the present invention can accurately detect different types of samples without interference from background signals.

### Example 2.3 Sensitivity Test

In this example, PEDV samples with different RNA concentrations were used for sensitivity test of the biological detection chip of the present invention. The RNA concentration of the PEDV stock solution was quantified by qPCR and then subjected to serial dilution. The test samples included quality control (negative control, buffer only), 1.5x10² copies/µL of PEDV (Sample 1), 1.5x10⁵ copies/µL of PEDV (Sample 2), and 1.5x10⁸ copies/µL of PEDV (Sample 3). Each sample was tested by the biological detection chip. The experimental procedure was the same as described in Example 2.1.

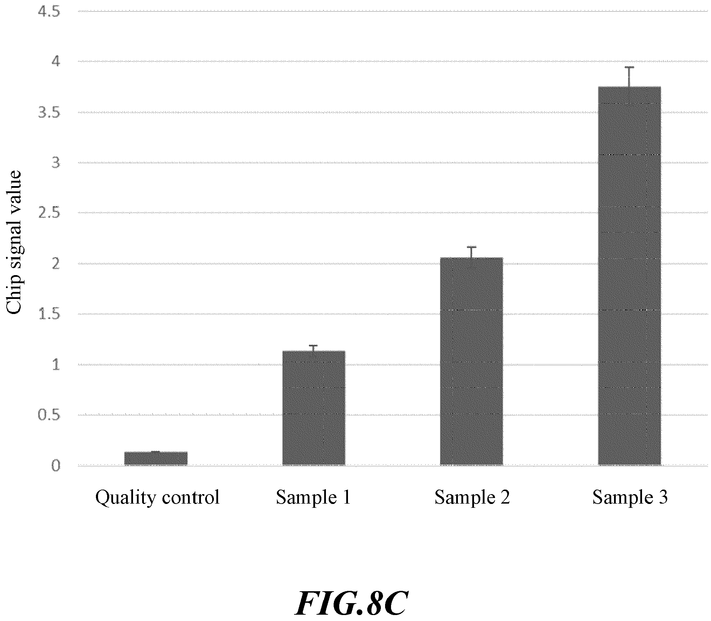

The test results are shown in FIG. 8C. The biological detection chip used in this example can detect signals at a concentration of at least 1.5x10² copies/µL of PEDV (Sample 1), demonstrating a high sensitivity of the chip. Moreover, the signal values increase in a concentration-dependent manner (i.e., Sample 3 > Sample 2 > Sample 1), indicating a potentially quantitative effect of the biological detection chip of the present invention.

### Example 3 Detection of Cortisol

In this example, the biological target is cortisol. APTES-Biotin modification method was used to modify the surface of the biological detection chip in this example, and a cortisol-specific antibody (Anti-Cortisol antibody [F4P1A3], Abcam, Cambridge, UK) was used as the biological probe.

The test samples included quality control (negative control, buffer only), 0.1 µg/dL cortisol (Sample 1), and 6 µg/dL cortisol (Sample 2). These test samples were prepared for examining the detection efficacy of the biological detection chip for cortisol. The experimental procedure was the same as described in Example 1.1, except that in this example, the incubation time for the test samples was 15 minutes.

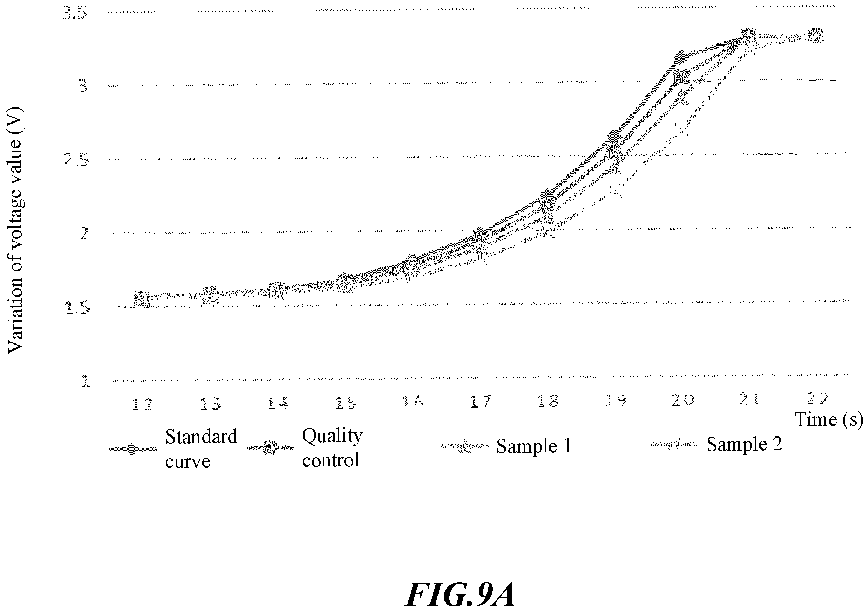

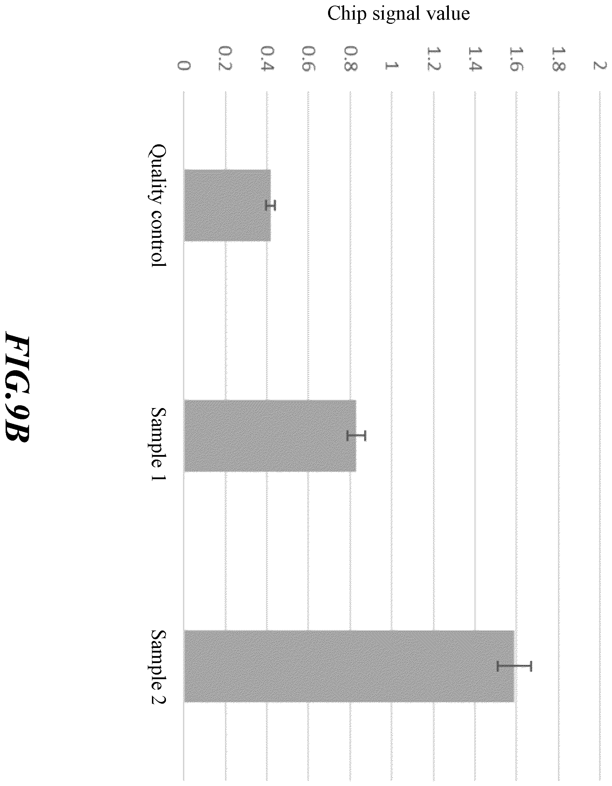

Please refer to FIGs. 9A and 9B for the experimental results. As shown in FIG. 9A, the electrical curves of the samples containing cortisol (Sample 1 and Sample 2) significantly deviate from the standard curve (baseline) in a concentration-dependent manner, indicating a potentially quantitative effect of the biological detection chip of the present invention. Furthermore, as shown in FIG. 9B, after quantifying the signals, the results show that the biological detection chip of the present invention detects the presence of cortisol at a concentration of at least 0.1 µg/dL in a test sample (Sample 1), demonstrating a high sensitivity of the chip.

In conclusion, based on the results of the examples, the biological detection chip of the present invention demonstrates the effectiveness in detecting PRRSV, PEDV, and cortisol. Compared to traditional detection processes, the biological detection chip of the present invention exhibits a high specificity, a high sensitivity, high accuracy, simplicity of process, ease of operation, rapid detection, and a potentially quantitative effect. The biological detection chip of the present invention can be used to effectively analyze biological phenomena, explore physiological activities and disease processes, and further develop treatment and/or improvement methods. It holds broad application prospects in the fields of life sciences, clinical diagnostics, and food safety.

Many changes and modifications in the above described embodiment of the invention can, of course, be carried out without departing from the scope thereof. Accordingly, to promote the progress in science and the useful arts, the invention is disclosed and is intended to be limited only by the scope of the appended claims.

## Claims

1. A biological detection chip, comprising a plurality of transistors disposed in an array having a plurality of rows and a plurality of columns, each of the plurality of transistors being disposed within one of the plurality of rows and one of the plurality of columns, and each of the plurality of transistors comprising:
a substrate layer comprising a shared source, a shared drain, and a channel area disposed between the shared source and the shared drain;
a floating gate disposed on the channel area and comprising a polysilicon oxide layer;
an extending metal connector disposed on the floating gate;
an extending gate disposed on the extending metal connector and electrically connected to the floating gate through the extending metal connector; and
a biological detection layer disposed on the extending gate and comprising an immobilization region and at least one biological probe immobilized on the immobilization region;
wherein a plurality of the biological detection layers forms a plurality of biological detection areas in parallel on the surface of the biological detection chip; and
wherein the plurality of transistors disposed in a same row of the plurality of rows are connected in parallel.

2. The biological detection chip of claim 1, wherein the floating gates of two adjacent transistors disposed in a same column of the plurality of columns have approximately the same distances to a shared source or a shared drain between the two adjacent transistors.

3. The biological detection chip of claim 1, wherein the floating gates of two adjacent transistors disposed in a same column of the plurality of columns have approximately the same distances overlapping a shared source or a shared drain between the two adjacent transistors.

4. The biological detection chip of any one of claims 1 to 3, wherein the immobilization region is generated through a surface modification process on the surface of the extending gate, wherein the surface modification process is selected from the group consisting of 3-aminopropyltrimethoxysilane-Glutaraldehyde (APTES-GA) modification method, 3-aminopropyltrimethoxysilane-N-hydroxysuccinimide (APTES-NHS) modification method, 3-aminopropyltrimethoxysilane-Biotin (APTES-Biotin) modification method, and 3-glycidoxypropyltrimethoxysilane (GPTMS) modification method.

5. The biological detection chip of any one of claims 1 to 4, wherein the immobilization region comprises a plurality of chemical structure selected from the group consisting of

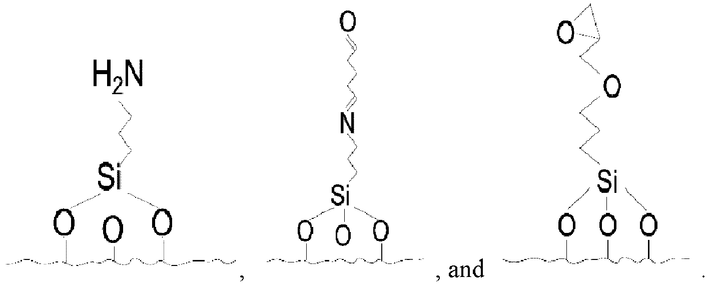

6. The biological detection chip of any one of claims 1 to 5, wherein the biological probe comprises at least one of deoxyribonucleic acid, ribonucleic acid, antibody, and aptamer.

7. The biological detection chip of any one of claims 1 to 6, wherein the plurality of biological detection areas comprises a micro-flow channel or a sample tank.

8. The biological detection chip of any one of claims 1 to 7, wherein the biological detection chip is for detecting porcine reproductive and respiratory syndrome virus (PRRSV), and the biological probe comprises at least one of synthetic oligonucleotides of SEQ ID NO: 1 to SEQ ID NO: 11.

9. The biological detection chip of any one of claims 1 to 7, wherein the biological detection chip is for detecting porcine epidemic diarrhea virus (PEDV), and the biological probe comprises at least one of synthetic oligonucleotides of SEQ ID NO: 12 to SEQ ID NO: 15.

10. The biological detection chip of any one of claims 1 to 7, wherein the biological detection chip is for detecting cortisol, and the biological probe is an anti-cortisol antibody.

11. A method for detecting a biological target, comprising the following steps of:
providing a biological detection chip of claim 1;
placing a blank sample in the plurality of biological detection areas connected in parallel, and measuring a gate voltage and a drain current of the plurality of transistors to establish a standard line;
placing a test sample in the plurality of biological detection areas for a predetermined time;
washing the plurality of biological detection areas by the blank sample;
measuring the gate voltage and the drain current of the plurality of transistors to establish a measurement line; and
comparing the measurement line with the standard line to determine whether the test sample contains the biological target.

12. The biological detection method of claim 11, wherein the biological target is porcine reproductive and respiratory syndrome virus (PRRSV), and the biological probe comprises at least one of synthetic oligonucleotides of SEQ ID NO: 1 to SEQ ID NO: 11.

13. The biological detection method of claim 11, wherein the biological target is porcine epidemic diarrhea virus (PEDV), and the biological probe comprises at least one of synthetic oligonucleotides of SEQ ID NO: 12 to SEQ ID NO: 15.

14. The biological detection method of claim 11, wherein the biological target is cortisol, and the biological probe is an anti-cortisol antibody.
